# EUROPEAN PATENT SPECIFICATION

(11) **EP 1 595 536 B1**
(45) Date of publication and mention of the grant of the patent: **06.01.2010**
(21) Application number: 03815939.8
(22) Date of filing: 21.02.2003
(51) Int. Cl.: A61K 31/122, A61P 31/00, A61P 7/00, C07C 50/32, A61P 35/00

(54) **PHARMACEUTICALS COMPRISING SHIKONINS AS ACTIVE CONSTITUENT**
ARZNEIMITTEL MIT SHIKONIN ALS WIRKSTOFF
PREPARATIONS PHARMACEUTIQUES DONT LA SHIKONINE EST LE PRINCIPE ACTIF

(43) Date of publication of application: 16.11.2005
(73) Proprietor: Beijing JBC Chinese Traditional Medicine Science and Technology Develop Co. Ltd., Beijing 100012 (CN)
(72) Inventor: WANG, Feixin, 100012 Beijing (CN)
(74) Representative: Barton, Matthew Thomas
(86) International application number: PCT/CN2003/000138
(87) International publication number: WO 2004/073699

(56) References cited:
- CN-A- 1 116 923
- CN-A- 1 117 525
- CN-A- 1 253 972
- DATABASE BIOSIS [Online] BIOSCIENCES INFORMATION SERVICE, PHILADELPHIA, PA, US; 1988, WANG W J ET AL: "A STUDY ON ABSORPTION DISTRIBUTION AND EXCRETION OF TRITIATED SHIKONIN IN MICE" XP002435685 Database accession no. PREV198886050680 & YAOXUE XUEBAO, vol. 23, no. 4, 1988, pages 246-251, ISSN: 0513-4870
- SHEN CHIEN-CHANG ET AL: "Antimicrobial activities of naphthazarins from Arnebia euchroma." JOURNAL OF NATURAL PRODUCTS DEC 2002, vol. 65, no. 12, December 2002 (2002-12), pages 1857-1862, XP002432671 ISSN: 0163-3864
- GADDIPATI JAYA P ET AL: "Inhibition of growth and regulation of IGFs and VEGF in human prostate cancer cell lines by shikonin analogue 93/637 (SA)" ANTICANCER RESEARCH, vol. 20, no. 4, July 2000 (2000-07), pages 2547-2552, XP009083260 ISSN: 0250-7005
- LIN Z-P ET AL: "SCREENING VALUABLE GENES FROM WILD SPECIES OF PLANTS" ADAMS, R. P. AND J. E. ADAMS (ED.). CONSERVATION OF PLANT GENES: DNA BANKING AND IN VITRO BIOTECHNOLOGY; PAPERS FROM THE ORGANIZATIONAL MEETING OF DNA BANK-NET, LONDON, ENGLAND, UK, APRIL 16-17, 1991. XII+345P. ACADEMIC PRESS, INC.: SAN DIEGO, CALIFO, 1992, pages 241-246, XP000671280 ISSN: 0-12-044140-3
- DATABASE MEDLINE [Online] US NATIONAL LIBRARY OF MEDICINE (NLM), BETHESDA, MD, US; October 1991 (1991-10), GUO X P ET AL: "[Clinical trial on the effects of shikonin mixture on later stage lung cancer]" XP002435686 Database accession no. NLM1806305 & ZHONG XI YI JIE HE ZA ZHI = CHINESE JOURNAL OF MODERN DEVELOPMENTS IN TRADITIONAL MEDICINE / ZHONGGUO ZHONG XI YI JIE HE YAN JIU HUI (CHOU), ZHONG YI YAN JIU YUAN, ZHU BAN OCT 1991, vol. 11, no. 10, October 1991 (1991-10), pages 598-599 , 580, ISSN: 0254-9034
- KONOSHIMA T ET AL: "STUDIES ON INHIBITORS OF SKIN TUMOR PROMOTION VI. INHIBITORY EFFECTS OF QUINONES ON EPSTEIN-BARR VIRUS ACTIVATION" JOURNAL OF NATURAL PRODUCTS (LLOYDIA), vol. 52, no. 5, 1989, pages 987-995, XP009083353 ISSN: 0163-3864
- SANKAWA U ET AL: "ANTI TUMOR ACTIVITY OF SHIKONIN AND ITS DERIVATIVES" CHEMICAL AND PHARMACEUTICAL BULLETIN (TOKYO), vol. 25, no. 9, 1977, pages 2392-2395, XP001536310 ISSN: 0009-2363
- YOON YOOSIK ET AL: "Shikonin, an ingredient of Lithospermum erythrorhizon induced apoptosis in HL60 human premyelocytic leukemia cell line" PLANTA MEDICA, vol. 65, no. 6, August 1999 (1999-08), pages 532-535, XP009803378 ISSN: 0032-0943
- MIN BYUNG SUN ET AL: "Cytotoxicity of shikonin metabolites with biotransformation of human intestinal bacteria" JOURNAL OF MICROBIOLOGY AND BIOTECHNOLOGY, vol. 10, no. 4, August 2000 (2000-08), pages 514-517, XP009083379 ISSN: 1017-7825
- HASHIMOTO SACHIKO ET AL: "beta-Hydroxyisovalerylshikonin is a novel and potent inhibitor of protein tyrosine kinases" JAPANESE JOURNAL OF CANCER RESEARCH, vol. 93, no. 8, August 2002 (2002-08), pages 944-951, XP009083380 ISSN: 0910-5050
- DATABASE BIOSIS [Online] BIOSCIENCES INFORMATION SERVICE, PHILADELPHIA, PA, US; 1993, YAMASAKI KATSUHIRO ET AL: "Screening test of crude drug extract on anti-HIV activity" XP002435687 Database accession no. PREV199497168920 & YAKUGAKU ZASSHI, vol. 113, no. 11, 1993, pages 818-824, ISSN: 0031-6903
- DATABASE MEDLINE [Online] US NATIONAL LIBRARY OF MEDICINE (NLM), BETHESDA, MD, US; September 1993 (1993-09), UEBA N ET AL: "[Anti-HIV-1 activities of crude drug, shikon (Lithospermi radix), in vitro]" XP002435688 Database accession no. NLM8271384 & NIPPON RINSHO. JAPANESE JOURNAL OF CLINICAL MEDICINE SEP 1993, vol. 51 Suppl, September 1993 (1993-09), pages 207-212, ISSN: 0047-1852
- MIN BYUNG-SUN ET AL: "Inhibitory effects of quinones on RNase H activity associated with HIV-1 reverse transcriptase." PHYTOTHERAPY RESEARCH : PTR MAR 2002, vol. 16 Suppl 1, March 2002 (2002-03), pages S57-S62, XP009083381 ISSN: 0951-418X
- AFZAL M ET AL: "SHIKONIN DERIVATIVES V. CHEMICAL INVESTIGATIONS OF ARNEBIA-DECUMBENS" AGRICULTURAL AND BIOLOGICAL CHEMISTRY, vol. 50, no. 3, 1986, pages 759-760, XP009083269 ISSN: 0002-1369
- HONDA G ET AL: "ISOLATION OF DEOXYSHIKONIN, AN ANTIDERMATOPHYTIC PRINCIPLE FROM LITHOSPERMUM ERYTHRORHIZON CELL CULTURES" JOURNAL OF NATURAL PRODUCTS, vol. 51, no. 1, 1988, pages 152-154, XP000672274 ISSN: 0163-3864
- AFZAL M ET AL: "SHIKONIN DERIVATIVES PART VI. CHEMICAL INVESTIGATIONS OF ARNEBIA-DECUMBENS" AGRICULTURAL AND BIOLOGICAL CHEMISTRY, vol. 50, no. 6, 1986, pages 1651-1652, XP009083268 ISSN: 0002-1369
- COULADOUROS E A ET AL: "Asymmetric Synthesis of Alkannin and Shikonin" TETRAHEDRON LETTERS, ELSEVIER, AMSTERDAM, NL, vol. 38, no. 41, 13 October 1997 (1997-10-13), pages 7263-7266, XP004091737 ISSN: 0040-4039
- LIN, ZHIBIN ET AL.: 'Study on anti-inflammatory action of chemical composition of shikonin' BEIJING YIXUEYUAN XUEBAO vol. 12, no. 2, 1980, pages 101 - 105

## Description

### Technical Field

The present invention is involved in a medicament containing Shikonin compounds or its salt, specially the medicament containing Shikonin compounds or its salt as an active component to prevent and treat microorganism infection in human body, inflammation, tumour, hemorrhage, hematopathy, and autoimmune disease.

### Technical background

Shikonin compounds are known compounds reported in literatures (Lin Zhibin, et al, P101-105, Issue 2, Volume 12, 1980, JOURNAL OF BEIJING MEDICAL UNIVERSITY), which have the following general formula structure:

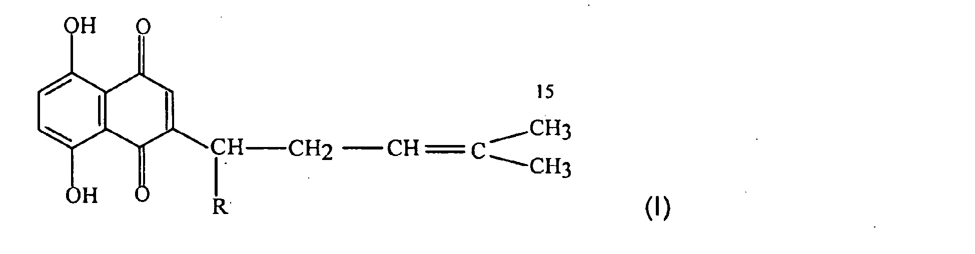

Shikonin compounds are insoluble in water but freely soluble in oil, alcohol or ethers, which are extracted from Boraginaceae plants such as lithospermum erythrorhizo Sieb.et zucc. and Arnebia euchroma(Royle)Johnst.. It's known that the extracted mixture has some functions such as anti-inflammation, but it has not been reported yet that the extracted Shikonin compounds and artificial or biosynthetic Shikonin compounds are used to manufacture medicaments in single compound or combination of several compounds, particularly for prevention and treatment of microorganism infection in human body, inflammation, tumour, hemorrhage, hematopathy and autoimmune disease.

Database Biosis (Online) Biosciences Information Service, Philadelphia, PA, US; 1993, Yamasaki Katsuhiro et al: "Screening test of crude drug extract on anti-HIV activity" discloses the anti-HIV-1 effects of 204 crude drugs of common use in Japan.

Min Byung-Sun Et Al: "Inhibitory effects of quinones on RNase H activity associated with HIV-1 reverse transcriptase." Phytotherapy Research: PTR MAR 2002. vol 16 Suppl 1, March 2002 (2202-03), pages S57-S62 discloses an in vitro assay method of ribonuclease H activity associated with reverse transcriptase from HIV-1. Shikonins were evaluated.

Honda G Et Al: "Isolation of Deoxyshikonin, an Antidermatophytic Principle from Lithospermum Erythrorhizon Cell Cultures" Journal of natural products, vol. 51, no.1 1988, pages 152-154 discloses the screening test of culture lines derived from plants to isolate active ingredients, including the isolation of deoxyshikonin.

Afzal M Et Al: "Shikonin Derivatives Part 1,7-13 VI.Chemical Investigations of Arnebia-Decumbens" Agricultural and Biological Chemistry, vol. 50, no. 6, 1986, pages 1651-1652 discloses extraction and evaluation of shikonin derivatives from plants.

Couladouros E A Et Al: "Asymmetric Synthesis of Alkannin and Shikonin" Tetrahedron Letters, Elsevier, Amsterdam, NL, vol. 38, no. 41, 13 October 1997 (1997-10-13), pages 7263-7266 discloses a new method for the synthesis of shikonin in 8 steps with 35% total yield.

According to the present invention, there is provided shikonin compounds represented by the following formula (I) or composition containing the same for use in the treatment of infection of mycoplasma pneumoniae, 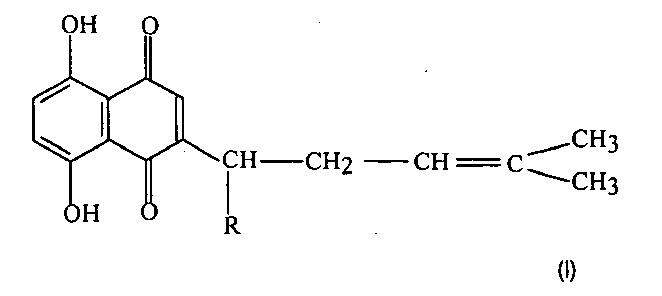 wherein, R is a group selected from H, OH, (CH₃)₂C=CHC(O)O-, CH₃C(O)O-, (CH₃)₂C=C(CH₃CH₂C(O)O-, (CH₃)₂COHCH₂C(O)O-, (CH₃)₂C[OC(O)CH₃]CH₂C(O)O-.

The present invention also provides shikonin compounds represented by the following formula (I) or composition containing the same for use in the treatment of infection of hepatitis B virus, 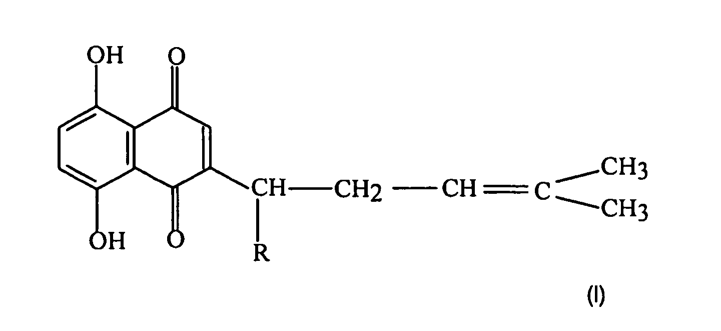 wherein, R is a group selected from H, OH, (CH₃)₂C=CHC(O)O-, CH₃C(O)O-, (CH₃)₂C=C(CH₃)CH₂C(O)O-(CH₃)₂COHCH₂C(O)O-, (CH₃)₂C[OC(O)CH₃]CH₂C(O)O-.

Preferably, R is a group selected from OH, (CH₃)₂C=CHC(O)O- and CH₃C(O)O-.

Advantageously, R is (CH₃)₂C=CHC(O)O- and/or CH₃C(O)O-.

Preferably, R is (CH₃)₂C=CHC(O)O-.

Conveniently, the purity of each mentioned shikonin compound or composition according to any one of claims 1-5, wherein the purity of each mentioned shikonin compound is 80% or higher.

Advantageously, the purity of each mentioned shikonin compound or composition according to claim 6, wherein the purity of each mentioned shikonin compound is 90% or higher.

Preferably, the composition of the invention contains a combination of 1-5 of the shikonin compounds in an amount of 70% or higher.

Advantageously, the composition further contains other active ingredients.

### Content of invention

Therefore, one aspect of the present disclosure is to provide a single compound or several compounds separated from the extracted mixture to manufacture a medicament for prevention and treatment of microorganism infection in human body, inflammation, tumor, hemorrhage, hematopathy and autoimmune disease.

The present disclosure provides a medicament to prevent and treat microorganism infection in human body, inflammation, tumour, hemorrhage, hematopathy, and autoimmune, which comprises one to five Shikonin compounds or its salt represented in the following Formula (1) 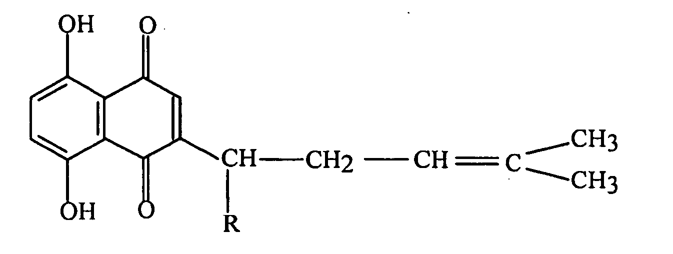 wherein, R is a group selected from a group composed of H(deoxyshikonin), OH(Shikonin), (CH₃)₂C=CHC(O)O-(β,β-dimethylacry), CH₃C(O)O-(Acetylshikonin), (CH₃)₂C=C(CH₃)CH₂C(O)O-(teracrylshikonin), (CH₃)₂COHCH₂C(O)-(β-hydroxyisovalerylshikonin), (CH₃)₂C[OC(O)CH₃]CH₂C(O)O-(β-acetoxyisovalerylshikonin); and preferably, said medicament contains 1 to 3 compounds selected from Shikonin, β, β-dimethylacrylshikonin and Acetylshikonin; more preferably, said medicament contains β,β-dimethylacrylshikonin and/or Acetylshikonin; the most preferably, said medicament containsβ, β-dimethylacrylshikonin. The salts of Shikonin compounds in this invention include the salts of alkali metals, alkaline earth metal and ammonium , etc thereof.

The medicament according to this disclosure contains one or more compound(s) of the Shikonin compounds as raw medicaments, of which the purity of single compound is 80% or more, and the perferred purity is 90% or more. When the medicine contains a combination of several compounds, the effective components thereof is 70% or more.

If necessary, the medicament can further contain other active components: There is no extraordinary restriction to the other active components, which the technologist can select properly in accordance to the existing technology.

The amount of Shikonin compounds contained in the medicament is in the range from 0.0001% to 75% (weight percent), which can be selected properly according to different preparation as well as symptoms of disease. When being used in human body, the daily dosage of the Shikonin compounds can be controlled between 10µg-20g, perferably 100µg-10g, more perferably 1 mg-8g, and the best one is 5g, which can be selected properly in accordance to the different status such as age, weight and symptoms of the subjects. It can be used for a single time or several times. The medicament can be delivered in oral administration, external application, injection, inhalation or skin penetration.

The Shikonin compounds can be used for prevention and treatment of microorganism infection including pathogenic Gram-positive micrococcus, such as staphylococcus, streptococcus pneumonia, staphylococcus epidermidis and enterococcus; pathogenic Gram-negative micrococcus such as Klebsiella pneumonia ozaenae, Serratia marcesens, Stenotrophomonas maltophilia; anaerobic or little aerobic pathogen such as Helicobacter pylori; Eumycetes such as deep and superficial eumycetes; Leuconostoc spp, aspergillus fumigatus, cryptococcus, dermatophyte, krusei leuconostoc spp, Cercospora punicae, etc; and all kinds of mycoplasma infection particularly the mycoplasma infection of the respiratory system; virus such as hepatitis B virus, cold virus, herpes virus and HIV virus, etc.

The Shikonin compounds can be used for prevention and treament of inflammation of human body, including phlebitis, vascular purpura, colpitis and edema, etc.

It also can be used for prevention and treatment of hemorrhage and hematopathy in human body, for instance, burning, scalding, all kinds of dermatitis, serticemia hemophilia, primary thrombocythemia, leukaemia, etc.

It also can be used for prevention and treatment of tumour especially malignant tumors, for instance, ascitic type tumour: liver cancer, L1210; solid tumour: W256, S180, gastric cancer 823, squamous cell carcinoma 109, Lewis lung cancer, etc.

The medicament containing Shikonin compounds can be used for prevention and treatment of autoimmune disease of human body, i.e. promoting human body's functions of nonspecific immunity and idiosyncratic cell-mediated immunity through improving the function of immune response of T lymphocytes.

Therefore, the medicament according to the present disclosure is available for respiratory system, digestive system, urinary system, reproductive system, blood system, circulating system and skin or mucous membrane in human body.

### Mode of carrying out the invention

The following text gives detailed description on the manufacture of pharmaceutical preparation containing Shikonin compounds and pharmacodynamics experiments of the present invention, but the protection scope of the present invention is not limited to this.

### Preparation example 1

Extracting the shattered 2 kg Arnebia euchroma(Royle)Johnst. with petroleum ether till residue of Arnebia euchroma(Royle)Johnst was colorless, recovering the solvent and obtaining 80 g dark red paste. Separating the paste through silica gel H-column liquid chromatography by gradiently elution with 1%-20% ethyl acetate-petroleum ether, and obtaining 7 compounds of Shikonin compounds as mentioned above, i.e. 2.944 g deoxyshikonin (yield is 3.68%), 0.712 g Shikonin (yield is 0.89%), 29.024 gβ, β -dimethylacrylShikonin (yield is 36.28%), 13.27 g Acetylshikonin(yield is 16.59%), 6.032 g teracrylshikonin (yield is 7.54%), 0.776 gβ-hydroxyisovaleryshikonin(yield is 0.97%), 0.792 gβ-acetoxyisovalerylshikonin(yield is 0.99%). The Analysis of high-pressure liquid chromatography shows that purity of all compounds is over 90%.

### Preparation example 2

2 kg Arnebia euchroma(Royle)Johnst.was shattered, and went through 20-40 meshes, and 70 g red ointment by CO₂-supercritical extraction was obtained. Separating the red ointment by high-pressure liquid preparative chromatography (Germany Knauer K1001 type) with preparative column: silica gel H 10µm 50X300mm and gradient elution with 1%-20% ethyl acetate-petroleum ether, and then obtaining 7 red compounds of Shikonin compounds as mentioned above, i.e. 3.486 g deoxyshikonin(yield is 4.98%), 0.707 g Shikonin (yield is 1.01%), 30.877 g β,β-dimethylacrylshikonin (yield is 44.11%), 15.869 g Acetylshikonin (yield is 22.67%), 6.034 g teracrylshikonin (yield is 8.62%), 0.91 g β -hydroxyisovalerylshikonin (yield is 1.30 %) and 0.77 g β -acetoxyisovalerylshikonin(yield is 1.10%). The Analysis of high-pressure liquid chromatography shows that purity of all compounds is over 90%.

### Example 1

Manufacturing troche with single or combination of several compounds of the obtained 7 compounds according to the method known in the art, of which the troche with 10% -70% Shikonin compounds was made according to actual demand.

Taking 100 g β, β-dimethylacrylshikonin obtained in Preparation example 1 or Preparation example 2, 100 g nucleated fiber, 30 g magnesium stearate, and 4 g hydroxypropyl methyl cellulose under the aseptic operation conditions, and 0.5 g tablets were made.

### Example 2

Manufacturing 0.5 g tablets with 100 g combination of Shikonin compounds obtained in Preparation example 1 or Preparation example 2 (combination proportion of Shikonin, β, β-dimethylacrylshikonin and Acetylshikonin was 1:1:2) as described in Example 1.

### Example 3

Manufacturing ointment of the above 7 Shikonin compounds according to the way widely known by technical personnel of the field, of which the ointment with 0.0001%-10% Shikonin compounds were made according to actual demand. Under the aseptic operation conditions, taking 0.5 g Shikonin compounds obtained in Preparation example 1 or Preparation example 2 (combination proportion of deoxyshikonin, Shikonin,β, β-dimethylacrylshikonin, Acetylshikonin andβ-hydroxyisovalerylshikonin is 0.7:1:1:2:0.5), 80 g vaseline, 10 g liquid paraffin and 10 g anhydrous lanolin and equably triturate them into products in separate bags for external use. This ointment also can be made into patch for skin penetration in a way widely known by technical personnel of the field.

### Example 4

Manufacturing the injection of the above 7 Shikonin compounds according to the way widely known by technical personnel of the field. Under the aseptic operation conditions, taking 0.5 gβ, β-dimethylacrylshikonin obtained in Preparation example 1 or Preparation example 2, 400 ml propylene glycol, 100 ml ethanol, 20 ml tween-80 and 15 ml benzyl alcohol, making them fully dissolved and adding water up to 1,000 ml. After mixing well, bottling them to be injection product.

The following description is on test result of effect of the medicament containing Shikonin compounds.

### (1) Preparation of the medicament

Respectively taking 5.0 mg Shikonin, β, β-dimethylacrylshikonin, Acetylshikonin obtained in Preparation example 1 or Preparation example 2. Making the medicament dissolved in 1 ml DMSO. After diluting by 50 times with RPMI-1640 culture medium, separately packing them and further diluting them into the following concentration: 100, 50, 25, 12.5, 6.25, 3.125, 1.5625, 0.78125, 0.390625 (µg/m).

### (2) Sensitivity test of the medicament

Separately packing the medicament at above concentration into the orifice plate and vaccinate with all bacterial strains at a density of 10³-10⁶.

The test result indicates that Shikonin, β, β -dimethylacrylshikonin and Acetylshikonin have high sensitivity to Gram-positive staphylococcus aureus and the MIC is 0.391-12.5µg/ml; for Gram-negative pathogen, the MIC of pneumobacillus is 0.391-6.25µg/ml and that of some bacterial strains is 12.5-50µg/ml; most isolates of bacillus prodigiosus and most bacterial strains of stenotrophomonas bacilli have a MIC of 0.391 - 3.125µg/ml. Therein, they are especially effective to stenotrophomonas bacilli and the MIC is 0.391-0.781µg/ml while that to berberine is 8-32µg/ml, i.e. it is obviously better than berberine. For bacteroid, especially bacteroides fragilis, the MIC is 0.391-6.25µg/ml; they are highly sensitive to Helicobacter pylori and the MIC is 0.391 -0.781 µg/ml.

Additionally, the result ofβ, β-dimethylacrylshikonin invitro antifungal test indicates that the MIC for candida and cryptococcus is 2.08-33.3µg/ml and MIC₉₀ is 33.3µg/ml; for fluconazole the MIC is 0.125-64µg/ml and MIC₉₀ is 69µg/ml; to dermatophyte the MIC is 4.16-8.32µg/ml with MIC₉₀ of 4.16µg/ml while the MIC of fluconazole to most bacterial strains of dermatophyte is 32-64µg/ml with MIC₉₀ of 64µg/ml. There are obvious differences in both of them. Furthermore, β, β-dimethylacrylshikonin has good inhibitory effect to C.krusei that resists fluconazole and the MIC is 8.32-16.6µg/ml, and for Pseudallescheria boydii that is insensitive to most antifungal medicaments like fluconazole, the MIC is 4.16-8.32µg/ml. Besides , the MIC of Acetylshikonin against cryptococcus neoformans is 3.90625 µg/ml, against red trichophyton is 0.90625- 62.5 µg/ml; the MIC of β, β-dimethylacrylshikonin against aspergillus fumigatus, cryptococcus and red trichophyton is 3.0625- 250µ g/ml. Therefore, Shikonin compounds are broadspectrum and effective antifungal drug.

In addition, Shikonin compounds of the invention have a MIC of over 200µg/ml on the microbes like Lactobacilli and Bifidobactirium beneficial for human body. Therefore, the above data indicates that medicaments with Shikonin compounds in the invention are were sensitive to pathogenic microorganism but insensitive to microbes beneficial for human body.

From the comparative experiment between the mixed extraction from Zicao and 1-3 kinds of Shikonin compounds, it is observed that the medicaments containing Shikonin compounds are obviously better than mixed extraction from Zicao; the results of MIC (µg/ml) are shown in Table 1.

**Table 1**

| Name of bacterial strain | A | B | C |
|---|---|---|---|
| Staphylococcus epidermidis | 12.5 | 0.391 | 0.7812 |
| Serratia marcescens | 25 | 0:781 | 3.125 |
| Bacteroid | >200 | 0.391 | 0.391 |
| Candida albicans | 500 | 3.9062 | 250 |

| | | | |
|---|---|---|---|
| Note: A is the mixed extraction B is β, β-dimethylacrylshikonin ; C is mixture of Shikonin compounds (the mixture ratio of Shikonin, β, β -dimethylacrylshikonin and Acetylshikonin is 1:1:2) | | | |

The experiment result of Shikonin, β, β-dimethylacrylshikonin and Acetylshikonin 's bacteriostatic effect on mycoplasma pneumoniae shows that, their MIC for mycoplasma pneumoniae are respectively 3.751 µg/ml, 2µg/ml and 7.819µg/ml, equivalent to the inhibitory effect of 0,1925µg/ml erythrocin.

The following table shows the test results for using Shikonin compound ointment made in Example 3 as external remedy for treating some disease.

**Table 2**

| Cases | Number of subjects | Effective percentage | Cured percentage | Days of treatment | Medicament Delivery route | Note |
|---|---|---|---|---|---|---|
| Burn & scalding | 300 | 100% | 100% | 6-20 | Direct delivery at affected part | 92 people scalded, 186 second degree superficial burns, 114 deep second degree and third degree burns |
| Hemorrhoids | 117 | 100% | 97.4% | 15 | Direct delivery at affected part | Recurrence in three cases after half a year |
| Herpes zoster | 98 | 100% | 100% | 3-7 | Direct delivery at affected part | Polyinosinic-pol ytidylin acid is used in 12 cases |
| Cervical erosion | 80 | 100% | 100% | 10-20 | Vagina delivery | |
| Children's nosebleed | 257 | 99.6% | 72.8% | 15 | Nasal cavity delivery | |
| Verruca plana | 100 | 96% | 81% | 10-30 | Direct delivery at affected part | |
| Chronic prostatitis | 40 | 82.5% | 57.5% | 10-20 | Anus delivery | |
| Acne | 50 | 92% | 60% | 15 | Direct delivery at affected part | |
| Bedsore | 30 | 100% | 100% | 7-21 | Direct delivery at affected part | |
| Eczema rhagadifor me | 98 | 94.9% | 66.3% | 10-30 | Direct delivery at affected part | |
| Verruca acuminata | 55 | 100% | 100% | 5-35 | Direct delivery at affected part | |
| Infantal diaper dermatitis | 208 | 100% | 100% | 2-6 | Direct delivery at affected part | |

It is observed from the above table that the external remedy of Shikonin compounds is suitable for treatment of most abscess, wound, scabies and herpes; the effect is prominent for burn and scalding without cicatrices after recovery.

Table 3 shows the animal test results for using Shikonin, β, β -dimethylacrylshikonin and Acetylshikonin to restrain tumor. (not part of the invention)

**Table 3**

| Type of tumour | β, β-dimethylacrylshikonin | | Acetylshikonin | | Shikonin | |
|---|---|---|---|---|---|---|
| | Tumor inhibitory rate | Life prolonged rate | Tumor inhibitory rate | Life prolonged rate | Tumor inhibitory rate | Life prolonged rate |
| Ascitic tyre liver cancer | | 113.4% | 47.8% | 112.6% | | 130.8% |
| S180 | 9.63% | | 35.7% | | | |
| Lewis lung cancer | 42.8% | | 52.6% | | | |
| L1210 | | | | 128% | | |
| W256 | | | 77% | | | |

It is observed from the above table that β, β-dimethylacrylshikonin has different extent of therapeutic effect for liver cancer, S180 and Lewis lung cancer; Acetylshikonin has different extent of therapeutic effect for liver cancer, S180, L1210 and Lewis lung cancer and W256; Shikonin is only effective for liver cancer.

As is shown in the experiment, using Shikonin compounds to inhibit virus, oral dosing β, β-dimethylacrylshikonin was made on the 7th day since ducks were infected by DHBV with 100mg/kg and twice a day, the inhibitory effect for DHBV-DNA level in blood serum of infected duck was prominent in 10 days (P<0.05-0.01) without toxic reaction; for the 50mg/kg group, significant inhibitory effect was shown (P<0.05).

As is shown in the invitro experiment using β, β-dimethylacrylshikonin to inhibit HBV at the concentration 30µg/ml, average inhibitory rate for HBsAg is 96.2601% and for HBeAg is 91.6056%.

Table 4 shows the invitro test results of Shikonin and β, β-dimethylacrylshikonin resisting HIV-1 reverse transcriptase and integrase.

**Table 4**

| | | IC₅₀(µg/ml) |
|---|---|---|
| Resisting HIV reverse transcriptase | Positive control PFA | 0.097 |
| | Shikonin compounds | >20 |
| Resisting HIV integrase | Positive control ABPS-Y | 0.922 |
| | Shikonin compounds | 12.467 |

The effect of Shikonin compounds on the model of mouse's low immunologic function caused by mitomycin C was tested. When 6mg/kg β, β-dimethylacrylshikonin was injected in the abdominal cavity for 5 days without interruption, the increase of cell toxicant in the mouse's splenic cell and NK cell was observed by around 20% (P<0.001), which indicates that β, β-dimethylacrylshikonin recovered the injury of intraperitoneal macrophage, improved the migration ability of intraperitoneal macrophage, raised the activity of T lymphocytes, and promoted the immune response of T lymphocytes, enhanced the nonspecific immunity and specific cell immunity effect of body.

## Claims

1. Shikonin compounds represented by the following formula (I) or composition containing the same for use in the treatment of infection of mycoplasma pneumoniae, 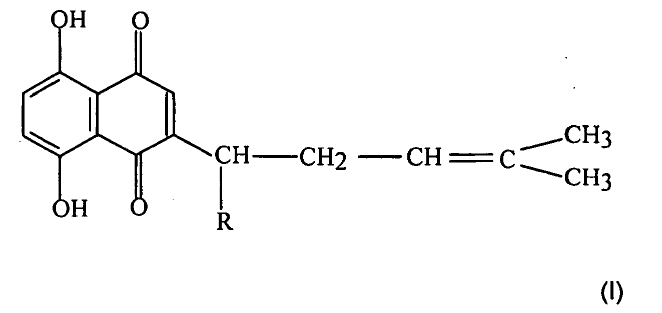 wherein, R is a group selected from H, OH, (CH₃)₂C=CHC(O)O-, CH₃C(O)O-, (CH₃)₂C=C(CH₃)CH₂C(O)O-,(CH₃)₂COHCH₂C(O)O-, (CH₃)₂C(OC(O)CH₃]CH₂C(O)O-.

2. Shikonin compounds represented by the following formula (I) or composition containing the same for use in the treatment of infection of hepatitis B virus, 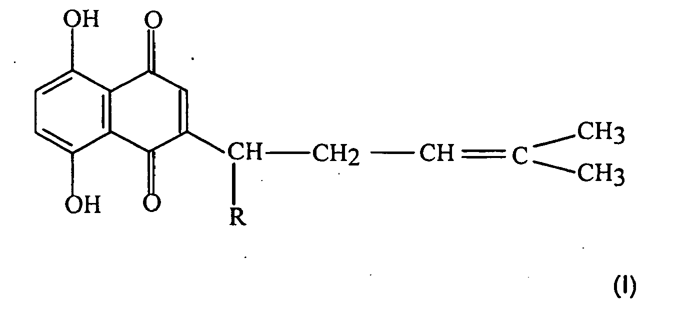 wherein, R is a group selected from H, OH, (CH₃)₂C=CHC(O)O-, CH₃C(O)O-, (CH₃)₂C=C(CH₃)CH_{Z}C(O)O-, (CH₃)₂COHCH₂C(O)O-, (CH₃)₂C(OC(O)CH₃]CH₂C(O)O-.

3. The shikonin compounds or composition according to claim 1 or 2, wherein R is a group selected from OH, (CH₃)₂C=CHC(O)O- and CH₃C(O)O-.

4. The shikonin compounds or composition according to claim 3, wherein R is (CH₃)₂C=CHC(O)O- and/or CH₃C(O)O-.

5. The shikonin compounds or composition according to claim 4, wherein R is (CH₃)₂C=CHC(O)O-.

6. The shikonin compounds or composition according to any one of claims 1-5, wherein the purity of each mentioned shikonin compound is 80% or higher.

7. The shikonin compounds or composition according to claim 6, wherein the purity of each mentioned shikonin compound is 90% or higher.

8. The composition according to any one of claims 1-7, which contains a combination of 1-5 the shikonin compounds in an amount of 70% or higher.

9. The composition according to any one of claims 1-8, which further contains other active ingredients.

## Patentansprüche

1. Shikoninverbindungen, die durch die folgende Formel (I) dargestellt werden, oder Zusammensetzung, die dieselben enthält, zur Verwendung bei der Behandlung einer Mykoplasma-Pneumonie-Infektion, 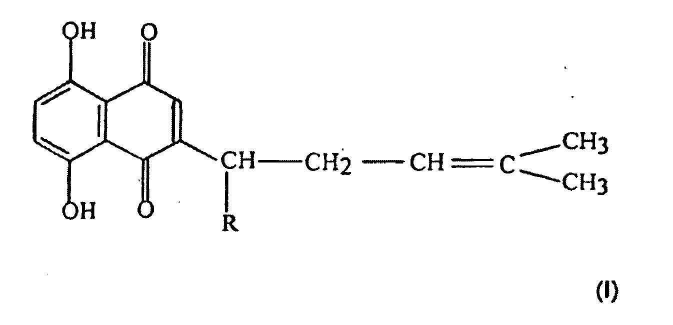 wobei R eine Gruppe ist, die aus Folgendem ausgewählt wird: H, OH, (CH₃)₂C=CHC(O)O-, CH₃C(O)O-, (CH₃)₂C=C(CH₃)CH₂C(O)O-, (CH₃)₂COHCH₂C(O)O-, (CH₃)₂C[OC(O)CH₃]CH₂C(O)O-.

2. Shikoninverbindungen, die durch die folgende Formel (I) dargestellt werden, oder Zusammensetzung, die dieselben enthält, zur Verwendung bei der Behandlung einer Hepatitis-B-Virusinfektion, 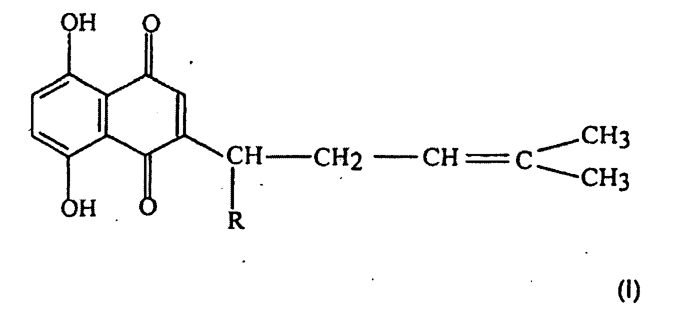 wobei R eine Gruppe ist, die aus Folgendem ausgewählt wird: H, OH, (CH₃)₂C=CHC(O)O-, CH₃C(O)O-, (CH₃)₂C=C(CH₃)CH₂C(O)O-, (CH₃)₂COHCH₂C(O)O-, (CH₃)₂C[OC(O)CH₃]CH₂C(O)O-.

3. Shikoninverbindungen oder Zusammensetzung nach Anspruch 1 oder 2, wobei R eine Gruppe ist, die aus Folgendem ausgewählt wird: OH, (CH₃)₂C=CHC(O)O- und CH₃C(O)O-.

4. Shikoninverbindungen oder Zusammensetzung nach Anspruch 3, wobei R (CH₃)₂C=CHC(O)O- und/oder CH₃C(O)O- ist.

5. Shikoninverbindungen oder Zusammensetzung nach Anspruch 4, wobei R (CH₃)₂C=CHC(O)O- ist.

6. Shikoninverbindungen oder Zusammensetzung nach einem der Ansprüche 1 bis 5, wobei die Reinheit jeder erwähnten Shikoninverbindung 80% oder mehr beträgt.

7. Shikoninverbindungen oder Zusammensetzung nach Anspruch 6, wobei die Reinheit jeder erwähnten Shikoninverbindung 90% oder mehr beträgt.

8. Zusammensetzung nach einem der Ansprüche 1 bis 7, die eine Kombination aus 1 bis 5 der Shikoninverbindungen in einer Menge von 70% oder mehr enthält.

9. Zusammensetzung nach einem der Ansprüche 1 bis 8, die des Weiteren andere aktive Inhaltsstoffe enthält.

## Revendications

1. Composés de shikonine représentés par la formule suivante (I) ou composition contenant ceux-ci destinée au traitement d'infections à mycoplasma pneumoniae, 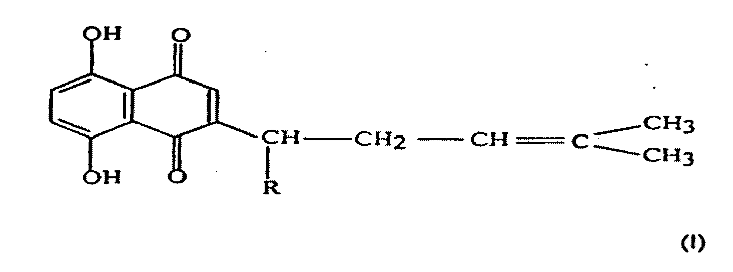 dans laquelle R est un groupe sélectionné parmi H, OH, (CH₃)₂C=CHC(O)O-, CH₃C(O)O-, (CH₃)₂C=C(CH₃)CH₂C(O)O-, (CH₃)₂COHCH₂C(O)O-, (CH₃)₂C[OC(O)CH₃]CH₂C(O)O-.

2. Composés de shikonine représentés par la formule suivante (I) ou composition contenant ceux-ci destinée au traitement d'infections du virus de l'hépatite B, 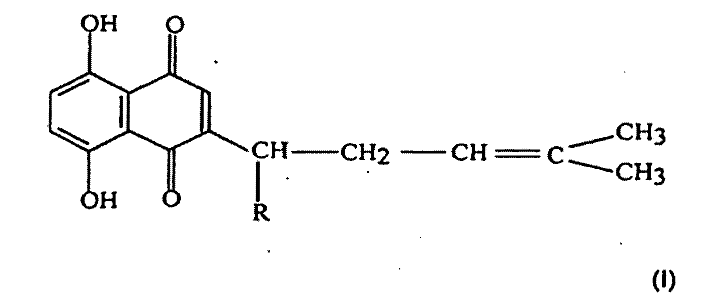 dans laquelle R est un groupe sélectionné parmi H, OH, (CH₃)₂C=CHC(O)O-, CH₃C(O)O-, (CH₃)₂C=C(CH₃)CH₂C(O)O-, (CH₃)₂COHCH₂C(O)O-, (CH₃)₂C[OC(O)CH₃]CH₂C(O)O-.

3. Composés de shikonine ou composition selon la revendication 1 ou 2, dans lesquels R est un groupe sélectionné parmi OH, (CH₃)₂C=CHC(O)O- et CH₃C(O)O-.

4. Composés de shikonine ou composition selon la revendication 3, dans lesquels R est (CH₃)₂C=CHC(O)O- et/ou CH₃C(O)O-.

5. Composés de shikonine ou composition selon la revendication 4, dans lesquels R est (CH₃)₂C=CHC(O)O-.

6. Composés de shikonine ou composition selon l'une quelconque des revendications 1 à 5, dans lesquels la pureté de chaque composé de shikonine mentionné est supérieure ou égale à 80 %.

7. Composés de shikonine ou composition selon la revendication 6, dans lesquels la pureté de chaque composé de shikonine mentionné est supérieure ou égale à 90 %.

8. Composition selon l'une quelconque des revendications 1 à 7, contenant une combinaison de 1 à 5 composés de shikonine dans une quantité supérieure ou égale à 70 %.

9. Composition selon l'une quelconque des revendications 1 à 8, contenant en outre d'autres ingrédients actifs.
